(19) Europäisches Patentamt
European Patent Office
Office européen des brevets

(11) Veröffentlichungsnummer: **0 001 428**
**B1**

# (12) EUROPÄISCHE PATENTSCHRIFT

(45) Veröffentlichungstag der Patentschrift: **24.03.82**

(21) Anmeldenummer: **78100984.0**

(22) Anmeldetag: **25.09.78**

(51) Int. Cl.³: **A 01 N 43/16,**
C 07 D 311/96,
C 07 D 491/10
//(C07D491/10, 311/00,
221/00)

## (54) Chromanderivate enthaltende insektizide und akarizide Mittel, ihre Herstellung und ihre Verwendung sowie Chromanderivate und ihre Herstellung.

(30) Priorität: **07.10.77 DE 2745305**

(43) Veröffentlichungstag der Anmeldung:
**18.04.79 Patentblatt 79/8**

(45) Bekanntmachung des Hinweises auf die Patenterteilung:
**24.03.82 Patentblatt 82/12**

(84) Benannte Vertragsstaaten:
**BE CH DE FR GB NL**

(56) Entgegenhaltungen:
**DE - A - 2 535 338**
**DE - A - 2 639 671**

(73) Patentinhaber: **BAYER AG**
**Zentralbereich Patente, Marken und Lizenzen**
**D-5090 Leverkusen 1, Bayerwerk (DE)**

(72) Erfinder: **Kabbe, Hans-Joachim, Dr.**
**Walter-Flex-Strasse 16**
**D-5090 Leverkusen 1 (DE)**
Erfinder: **Widdig, Arno, Dr.**
**Eifgenstrasse 8**
**D-5068 Odenthal (DE)**
Erfinder: **Stendel, Wilhelm, Dr.**
**In den Birken 55**
**D-5600 Wuppertal 1 (DE)**
Erfinder: **Roessler, Peter, Dr.**
**Elsterstrasse 15**
**D-5060 Bergisch Gladbach (DE)**

**EP 0 001 428 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Chromanderivate enthaltende insektizide und akarizide Mittel, ihre Herstellung und ihre Verwendung sowie Chromanderivate und ihre Herstellung

Die Erfindung betrifft die Verwendung von teilweise bekannten Chromanderivaten als Insektizide und Akarizide, ferner bestimmte Chromanderivate und deren Herstellung.

Es ist bekannt, daß einige in der Natur vorkommende Chromene, z.B. Precocen I und Precocen II, eine entwicklungsinhibitorische Wirkung bei Insekten zeigen und deshalb als Insektizide wirken können. (vgl. Chem. Eng. News *1976*, Heft 16, Seite 19). Ihre Wirkung befriedigt jedoch vor allem bei niedrigen Aufwandkonzentrationen nicht immer.

Es wurde nun gefunden, daß die Chromanderivate der allgemeinen Formel I

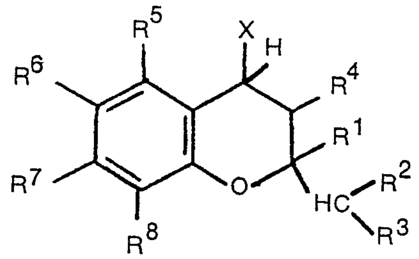

(I)

in welcher

$R^1$ bis $R^4$ gleich oder verschieden sind und für Wasserstoff, Carboxyl oder für Alkyl, Alkenyl oder Alkoxy-carbonyl mit jeweils bis zu 18 Kohlenstoffatomen, Cycloalkyl mit 3 bis 18 Kohlenstoffatomen, Aryl mit 6 bis 14 Kohlenstoffatomen oder Aralkyl mit 7 bis 18 Kohlenstoffatomen stehen, wobei diese Reste durch Halogen, Cyano, $C_{1-6}$-Alkyl, $C_{1-6}$-Alkoxy, $C_{2-6}$-Alkoxycarbonyl, $C_{3-6}$-Alkoxycarbonylalkyl, Amino, $C_{1-6}$-Alkylamino, $C_{2-6}$-Dialkylamino, Phenyl oder Carboxyl substituiert sein können und

$R^2$ zusätzlich für Amino, Pyrrolidinyl, Piperidinyl und Dialkylamino mit bis zu 18 Kohlenstoffatomen steht und

$R^1$ und $R^2$ gemeinsam mit den angrenzenden Kohlenstoffatomen carbocyclische Ringe der Reihe Cyclopropan, Cyclopentan, Cyclohexan, Cycloheptan, Cyclooctan, Cyclononan, Cyclodecan, Cycloundecan, Cyclododecan, Cyclohexen, Cycloocten, Cyclododecen und Tetralin oder heterocyclische Ringe der Reihe Piperidin, Pyrrolidin, Tetrahydrofuran, Tetrahydropyran, Tetrahydrothiopyran und $—CH_2—CH_2—N(CH_3)—CH_2—$ bilden können und

$R^5$ bis $R^8$ gleich oder verschieden sind und für Wasserstoff, Halogen, Hydroxy, Nitro, Cyano, Amino und Carboxy oder für Alkyl, Alkenyl, Alkoxy, Alkoxycarbonyl, Alkylamino, Dialkylamino mit jeweils bis zu 18 Kohlenstoffatomen, Pyrrolidinyl oder Piperidinyl, Cycloalkyl mit 3 bis 18 Kohlenstoffatomen, Aryl oder Aryloxy mit 6 bis 14 Kohlenstoffatomen und Aralkyl oder Aralkoxy mit 7 bis 18 Kohlenstoffatomen, wobei diese Reste substituiert sein können durch Halogen, $C_1—C_6$-Alkyl, $C_1—C_6$-Alkoxy, $C_2—C_6$-Alkoxycarbonyl, $C_3—C_6$-Alkoxycarbonylalkyl, Amino, $C_1—C_6$-Alkylamino und $C_2—C_6$-Dialkylamino, Phenyl oder Carboxyl oder für Formylamino, Acetylamino, Propionylamino, Valeroylamino und Benzoylamino stehen und

wobei 2 der Reste $R^5$ bis $R^8$, die in o-Stellung zueinander stehen, auch für Tetramethylen oder den Rest $—CH = CH—CH = CH—$ stehen können und

X für OH oder den Rest $—NR^9R^{10}$ steht, wobei

$R^9$ und $R^{10}$ gleich oder verschieden sind und für Wasserstoff, Alkyl oder Alkenyl mit bis zu 18 Kohlenstoffatomen, Cycloalkyl mit 3 bis 18 Kohlenstoffatomen, Aryl mit 6 bis 14 Kohlenstoffatomen, Aralkyl mit 7 bis 18 Kohlenstoffatomen oder gemeinsam mit dem Stickstoffatom, an welches sie gebunden sind, einen Pyrrolidin-, Piperidin-, Morpholin-, N-Methylpiperazin-, N-Phenylpiperazin-Ring bilden sowie die Salze der Verbindungen, in denen

X für den Rest $—NR^9R^{10}$ steht, sowie die Salze der Verbindungen, in denen X für den Rest $—NR^9R^{10}$ steht, starke, die Entwicklung von Insekten und Spinnentieren hemmende Eigenschaften aufweisen.

Überraschenderweise zeigen die Chromanderivate der allgemeinen Formel I erheblich bessere entwicklungshemmende Eigenschaften als die aus dem Stand der Technik bekannten Verbindungen ähnlicher Struktur und analoger Wirkungsrichtung. Die erfindungsgemäßen Wirkstoffe stellen somit eine Bereicherung der Technik dar.

Die erfindungsgemäßen Wirkstoffe sind zum Teil bekannt (vgl. DE—OS 2 639 671). Eine insektizide Wirkung dieser Verbindungen ist nicht bekannt.

Neue Verbindungen der allgemeinen Formel I, in der der Rest X für OH steht, werden erhalten durch Reduktion der entsprechenden bekannten Chromanone-(4) der allgemeinen Formel II,

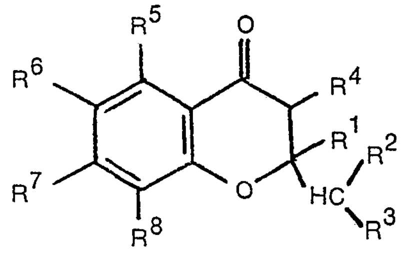

(II)

in welcher $R_1$ bis $R_8$ die oben angegebene Bedeutung besitzen.

Die Reduktion der Chromanone der allgemeinen Formel II zu den Chromanolen der allgemeinen Formel I, in welcher X für OH steht, kann man mit Metallhydriden oder mit Komplexen von Metallhydriden durchführen, z.B. mit Borhydrid, Natriumborhydrid, Lithiumaluminiumhydrid oder dem Boran/Dimethylamin-Komplex. Diese Reaktion wird vorzugsweise in einem gegen das betreffende Reduktionsmittel inerten Lösungsmittel durchgeführt, beispielsweise in Kohlenwasserstoffen (Benzol, Toluol), Äthern (Diäthyläther, Tetrahydrofuran, Dioxan), Alkoholen (Methanol, Äthanol, Glykolmonomethyläther) oder Gemischen dieser Lösungsmittel. Man arbeitet bei Temperaturen von −20 bis +100°C, vorzugsweise 0 bis 40°C (s. Wagner-Zook, Synthetic Organic Chemistry, S. 149, John Wiley a. Sons Inc., New York 1953). Die Reduktion kann auch mit Wasserstoff in Gegenwart von Hydrierkatalysatoren durchgeführt werden, insbesondere mit feinverteilten Schwermetallkatalysatoren wie Platin, Palladium, Nickel oder Kobalt (s. Wagner-Zook, Synthetic Organic Chemistry, S. 149, John Wiley a. Sons Inc., New York 1953). Die Hydrierung kann gegebenenfalls unter erhöhtem Druck und gegebenenfalls in Gegenwart eines Lösungsmittels erfolgen, z.B. von Kohlenwasserstoffen (Benzol, Toluol, Cyclohexan), Äthern (Diäthyläther, Dibutyläther, Tetrahydrofuran, Dioxan), Alkoholen (Methanol, Äthanol, Glykolmonomethyläther) oder Gemischen davon.

Neue Verbindungen der allgemeinen Formel I, in der der Rest X für $-NR^9R^{10}$ steht, erhältman, indem man

a) Chromanone-(4) der allgemeinen Formel II in Gegenwart von Aminen der allgemeinen Formel III

$$HNR^9R^{10} \qquad (III)$$

in welcher

$R^9$ und $R^{10}$ die oben angegebene Bedeutung besitzen, unter reduzierenden Bedingungen behandelt, oder

b) 4-Halogen- oder 4-Sulfonyloxychromane der allgemeinen Formel IV

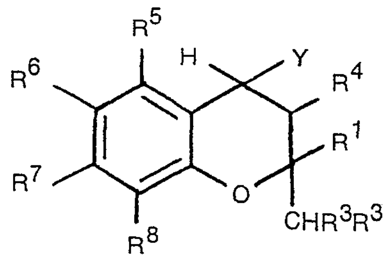

(IV)

in welcher

$R^1$ bis $R^8$ die oben angegebene Bedeutung besitzen und

Y für Halogen oder $-O-SO_2R^{11}$ steht, wobei $R^{11}$ für Alkyl, Cycloalkyl, Aralkyl oder Aryl steht mit Aminen der allgemeinen Formel III umsetzt, oder

c) 4-Aminochromane der allgemeinen Formel I in welcher X für $-NR^9R^{10}$ steht, wobei der Rest $R^{10}$ für Wasserstoff steht, mit einer Verbindung der allgemeinen Formel V

$$R^{10}-Y \qquad (V)$$

in welcher

$R^{10}$ und Y die oben angegebene Bedeutung besitzen, umsetzt oder

d) Verbindungen der allgemeinen Formel VI

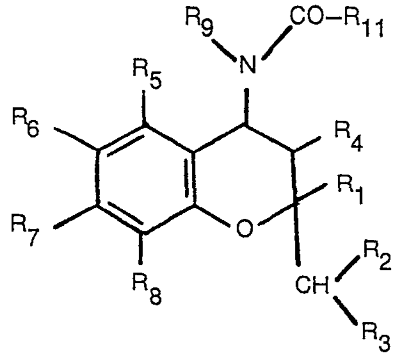

(VI)

in welcher

$R^1$ bis $R^9$ und $R^{11}$ die oben angegebene Bedeutung besitzen, mit Reductionsmitteln, insbesondere gemischten Metallhydriden, wie Lithiumaluminiumhydrid oder Natriumborhydrid, behandelt oder

e) 4-Aminochromane der allgemeinen Formel I, in der X für $—NR^9R^{10}$ steht und mindestens einer der Reste $R^9$ oder $R^{10}$ für Wasserstoff steht, mit Aldehyden der Formel VII

$$R_{12}—CHO \qquad (VII)$$

in der

$R_{12}$ für einen gegebenenfalls substituierten Alkyl-, Aryl- oder Aralkylrest steht, unter reduzierenden Bedingungen umsetzt.

4-Aminochromane der Formel I, in der X für $—NR^9R^{10}$ steht und $R^9$ und $R^{10}$ für Wasserstoff stehen, erhält man auch indem man 4-Oximinochromane der allgemeinen Formel VIII

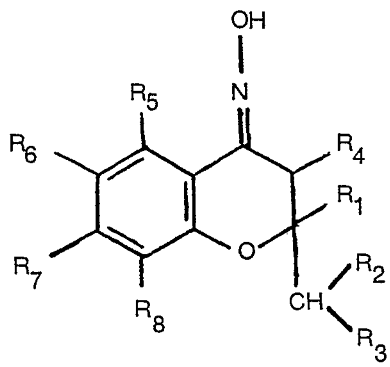

(VIII)

in welcher

$R^1$ bis $R^8$ die oben angegebene Bedeutung besitzt, reduziert.

Bevorzugte erfindungsgemäße Wirkstoffe der allgemeinen Formel I, in der $R^1$ bis $R^{10}$ jeweils einen Kohlenwasserstoffrest bedeutet, sind solche, in denen die Reste $R^1$ bis $R^{10}$ gleich oder verschieden sind und beispielsweise für geradkettige oder verzweigte Alkylreste oder Alkenylreste mit bis zu 12, besonders bevorzugt bis zu 6, Kohlenstoffatomen stehen. Als Alkyl- oder Alkenylreste seien beispielsweise genannt Methyl, Äthyl, Propyl, Isopropyl, Butyl, Isobutyl, tert.-Butyl, Hexyl, Nonyl, Decyl, Undecyl, Octadecyl, Buten-(3)-yl, 4-Methylpenten-(3)-yl, 4,8-Dimethylnonadien-(3,7)-yl. Ferner sind bevorzugt Wirkstoffe, in denen $R^1$ bis $R^{10}$ für Cycloalkylreste mit 4 bis 12, insbesondere bevorzugt mit 5 und 6, Kohlenstoffatomen, wie beispielsweise Cyclopropyl, Cyclobutyl, Cyclopentyl, Cyclohexyl, Cycloheptyl, Cyclooctyl, Cyclododecyl, Cycloheptadecyl und Cyclooctadecyl, bevorzugt Cyclopentyl und Cyclohexyl, ferner für Arylreste mit 6 bis 14 Kohlenstoffatomen, wie Phenyl, Naphthyl, Anthracenyl, bevorzugt Phenyl, ferner für Aralkylreste, deren aliphatischer Teil 1 bis 8, bevorzugt 1 bis 4, Kohlenstoffatome enthält und deren aromatischer Teil einen carbocyclischen Rest mit 6 bis 10 Kohlenstoffatomen darstellt, wie Benzyl, Phenyläthyl, Phenylpropyl, Phenylbutyl, Naphthylmethyl und Naphthyläthyl, bevorzugt Benzyl stehen.

Ferner sind bevorzugt Wirkstoffe, in denen $R^1$ mit $R^2$ unter Ausbildung eines Ringes verbunden sind, wobei als carbocyclische Reste Cyclopropan, Cyclopentan, Cyclohexan, Cycloheptan, Cyclooctan, Cyclononan, Cyclodecan, Cycloundecan, Cyclododecan, Cyclohexen, Cycloocten, Cyclododecen und Tetralin und als heterocyclische Ringe Piperidin, Pyrrolidin, Tetrahydrofuran, Tetrahydropyran und Tetrahydrothiopyran oder $—CH_2—CH_2—N(CH_3)—CH_2$ (vergleiche Beispiel 9) stehen.

Bei den bevorzugten Wirkstoffen entsprechen die Definitionen der Alkyl- bzw. Arylreste der Alkoxy-, Alkoxy-carbonyl-, Alkylamino-, Dialkylamino, Aryloxy- und der Aralkoxyreste hinsichtlich ihrer Kohlenstoffzahl dem vorstehend angegebenen Bedeutungsumfang.

Als bevorzugte Alkoxygruppen seien solche mit bis zu 4 Kohlenstoffatomen, wie Methoxy, Äthoxy, Propoxy, Isopropoxy, Butoxy, Isobutoxy und tert.-Butoxy, genannt.

Als bevorzugte Aryloxygruppen seien Phenoxy und Naphthoxy genannt.

Als bevorzugte Aralkoxygruppen seien solche mit 7 bis 10 Kohlenstoffatomen, wie Benzyloxy, Phenyläthoxy, Phenylpropoxy, Phenylisopropoxy, Phenylbutoxy, Phenylisobutoxy und Phenyl-tert.-butoxy, genannt.

Als bevorzugte Alkoxycarbonylgruppen seien solche mit bis zu 4 Kohlenstoffatomen im Alkylrest, wie Methoxycarbonyl, Äthoxycarbonyl, Propoxycarbonyl, Isopropoxycarbonyl, genannt.

Als bevorzugte Alkyl- und Dialkylaminogruppen seien solche mit bis zu 3 Kohlenstoffatomen im Alkylrest, wie Methylamino, Äthylamino, Isopropylamino, Dimethylamino, Diäthylamino, Dipropylamino und Diisopropylamino, genannt. Es ist auch möglich, daß die beiden Alkylreste der Dialkylaminogruppe zu einem Ring geschlossen sind, wie beispielsweise Pyrrolidinyl, Piperidinyl.

Die Acylaminogruppe bei dem Rest $R^{11}$ kann durch einen aliphatischen oder aromatischen Rest substituiert sein, wobei der aliphatische und der aromatische Rest den obengenannten Bedeutungsumfang haben. Als Acylaminogruppen seien beispielsweise genannt: Formylamino, Acetylamino, Propionylamino, Valeroylamino und Benzoylamino.

Als Halogene seien Fluor, Chlor, Brom und Jod, bevorzugt Chlor, genannt.

Besonders zu erwähnen seien folgende Wirkstoffe der allgemeinen Formel I:

2,2-Pentamethylenchromanol-(4)
2,2-Pentamethylen-7-hydroxychromanol-(4)
2,2-Pentamethylen-6-hydroxychromanol-(4)
2,2-Pentamethylen-6-methoxychromanol-(4)
2,2-Pentamethylen-7-methoxychromanol-(4)
7-Acetylamino-2,2-pentamethylenchromanol-(4)
6-Cyclohexyl-2,2-pentamethylenchromanol-(4)
5-Chlor-7-phenyl-2,2-pentamethylenchromanol-(4)
7-Alkoxy-2,2-pentamethylenchromanol-(4)
6-Äthoxycarbonylmethoxy-2,2-pentamethylenchromanol-(4)
6-Nitro-2,2-pentamethylenchromanol-(4)
5-Cyan-2,2-pentamethylenchromanol-(4)
7-Trifluormethyl-2,2-pentamethylenchromanol-(4)
6-Carboxy-2,2-pentamethylenchromanol-(4)
7-Methoxycarbonyl-2,2-pentamethylenchromanol-(4)
6-Butyramido-2,2-pentamethylenchromanol-(4)
7-Amino-2,2-pentamethylenchromanol-(4)
5-Hydroxy-7-phenyl-2,2-pentamethylen-chromanol-(4)
2-Methyl-2-($\gamma$-diäthylaminopropyl)-chromanol-(4)
2-Methyl-2-($\beta$-carboxyäthyl)-chromanol-(4)
2-Methyl-2-nonyl-7-hydroxy-chromanol-(4)
2-Methyl-2-($\beta$-N-pyrrolidinyläthyl-chromanol-(4)
2-Methyl-2-($\delta$-carboxybutyl)-chromanol-(4)
2,2-Tetramethylenchromanol-(4)
7-Hydroxy-2,2-tetramethylenchromanol-(4)
6-Hydroxy-2,2-tetramethylenchromanol-(4)
8-Methoxy-2,2-tetramethylenchromanol-(4)
6-Äthoxy-2,2-tetramethylenchromanol-(4)
7-Chlor-2,2-tetramethylenchromanol-(4)
5-Brom-2,2-tetramethylenchromanol-(4)
2-Isopropyl-3-phenyl-6-methyl-chromanol-(4)
2,3,6-Trimethyl-chromanol-(4)
5,7-Dihydroxy-2,2-tetramethylenchromanol-(4)
6,8-Dihydroxy-2,2-tetramethylenchromanol-(4)
5,8-Dihydroxy-2,2-tetramethylenchromanol-(4)
5,7,8-Trihydroxy-2,2-tetramethylenchromanol-(4)
7-Benzyloxy-2,2-tetramethylenchromanol-(4)
6-Dimethylamino-2-isopropyl-chromanol-(4)
7-Acetamino-2-isopropyl-chromanol-(4)
7-Chlor-2-propyl-chromanol-(4)
6-Hexylamino-2-methyl-2-nonyl-chromanol-(4)
5-Hydroxy-7-pentyl-2,2-tetramethylen-chromanol-(4)
5-Hydroxy-7-pentyl-2,2-undecamethylen-chromanol-(4)
5-Hydroxy-7-heptyl-2-methyl-2-nonyl-chromanol-(4)
5-Methyl-7-hydroxy-2-methyl-2-$\delta$-carboxybutyl-chromanol-(4)
6-Hydroxy-2-methyl-2-$\delta$-carboxybutyl-chromanol-(4)

7-Hydroxy-2-δ-carboxybutyl-chromanol-(4)
5-Hydroxy-7-pentyl-2-methyl-2-β-carboxyäthyl-chromanol-(4)
6-Hydroxy-2-methyl-2-β,β,β-trifluoräthyl-chromanol-(4)
2-Methyl-2-N-pyrrolidinylpropyl-chromanol-(4)
2-Methyl-2-benzyl-chromanol-(4)
2-Hydroxy-butyl-chromanol-(4)
6-Chlor-8-methyl-2,2-tetramethylenchromanol-(4)
6,8-Dichlor-2,2-pentamethylenchromanol-(4)
4-Methyl-amino-2,2-tetramethylenchroman
4-Dimethyl-amino-2,2-tetramethylenchroman
4-Äthyl-amino-2,2-tetramethylenchroman
4-Diäthyl-amino-2,2-tetramethylenchroman
4-Dipropyl-amino-2,2-tetramethylenchroman
4-Isopropyl-amino-2,2-tetramethylenchroman
4-Diisopropyl-amino-2,2-tetramethylenchroman
4-n-Butyl-amino-2,2-tetramethylenchroman
4-Di-n-butyl-amino-2,2-tetramethylenchroman
4-Isobutyl-amino-2,2-tetramethylenchroman
4-Diisobutyl-amino-2,2-tetramethylenchroman
4-tert.-Butyl-amino-2,2-tetramethylenchroman
4-(2'-Äthyl-hexyl)-amino-2,2-tetramethylenchroman
4-n-Hexyl-amino-2,2-tetramethylenchroman
4-Octyl-amino-2,2-tetramethylenchroman
4-Dodecyl-amino-2,2-tetramethylenchroman
4-Hexadecyl-amino-2,2-tetramethylenchroman
4-Allyl-amino-2,2-tetramethylenchroman
4-Diallyl-amino-2,2-tetramethylenchroman
4-(2-Methoxyäthyl)-amino-2,2-tetramethylenchroman
4-(2-Äthoxyäthyl)-amino-2,2-tetramethylenchroman
4-(3-Methoxypropyl)-amino-2,2-tetramethylenchroman
4-(2-Cyanäthyl)-amino-2,2-tetramethylenchroman
4-(3-Cyanäthyl)-amino-2,2-tetramethylenchroman
4-(2-Phenoxyäthyl)-amino-2,2-tetramethylenchroman
4-(3-Phenoxypropyl)-amino-2,2-tetramethylenchroman
4-Cyclopropyl-amino-2,2-tetramethylenchroman
4-Cyclobutyl-amino-2,2-tetramethylenchroman
4-Cyclopentyl-amino-2,2-tetramethylenchroman
4-Cyclohexyl-amino-2,2-tetramethylenchroman
4-Dicyclohexyl-amino-2,2-tetramethylenchroman
4-Cyclooctyl-amino-2,2-tetramethylenchroman
4-Cyclododecyl-amino-2,2-tetramethylenchroman
4-Phenyl-amino-2,2-tetramethylenchroman
4-(2-Chlorphenyl)-amino-2,2-tetramethylenchroman
4-(3-Chlorphenyl)-amino-2,2-tetramethylenchroman
4-(4-Chlorphenyl)-amino-2,2-tetramethylenchroman
4-(2,4-Dichlorphenyl)-amino-2,2-tetramethylenchroman
4-(3,4-Dichlorphenyl)-amino-2,2-tetramethylenchroman
4-(2-Methyl-phenyl)-amino-2,2-tetramethylenchroman
4-(3-Methyl-phenyl)-amino-2,2-tetramethylenchroman
4-(4-Methyl-phenyl)-amino-2,2-tetramethylenchroman
4-(2,4-Dimethylphenyl)-amino-2,2-tetramethylenchroman
4-(3,4-Dimethylphenyl)-amino-2,2-tetramethylenchroman
4-(4-Methoxyphenyl)-amino-2,2-tetramethylenchroman
4-(3-Äthoxyphenyl)-amino-2,2-tetramethylenchroman
4-(2-Isopropoxyphenyl)-amino-2,2-tetramethylenchroman
4-(1-Naphthyl)-amino-2,2-tetramethylenchroman
4-(2-Naphthyl)-amino-2,2-tetramethylenchroman
4-[1-(4-Chlornaphthyl)]-amino-2,2-tetramethylenchroman
4-[1-(4-Brom-naphthyl)]-amino-2,2-tetramethylenchroman
4-Benzyl-amino-2,2-tetramethylenchroman
4-Phenyläthyl-amino-2,2-tetramethylenchroman
4-Phenylpropyl-amino-2,2-tetramethylenchroman
4-($\Delta^2$-Cyclohexenyl)-amino-2,2-tetramethylenchroman
4-($\Delta^3$-Cyclohexenyl)-amino-2,2-tetramethylenchroman
4-(4-Chlor-benzyl)-amino-2,2-tetramethylenchroman

4-(4-Brombenzyl)-amino-2,2-tetramethylenchroman
4-(4-Methoxybenzyl)-amino-2,2-tetramethylenchroman
4-(4-Methylbenzyl)-amino-2,2-tetramethylenchroman
4-(Naphthyl-1-methyl)-amino-2,2-tetramethylenchroman
4-(Naphthyl-2-methyl)-amino-2,2-tetramethylenchroman
4-(4-Bromphenyläthyl)-amino-2,2-tetramethylenchroman
4-(4-Methoxyphenyläthyl)-amino-2,2-tetramethylenchroman
4-(4-Chlor-phenylpropyl)-amino-2,2-tetramethylenchroman
4-(4-Methoxyphenylpropyl)-amino-2,2-tetramethylenchroman
4-Pyrrolidino-2,2-tetramethylenchroman
4-Piperidino-2,2-tetramethylenchroman
4-(4-Methylpiperidino)-2,2-tetramethylenchroman
4-(2,4,6-Trimethylpiperidino)-2,2-tetramethylenchroman
4-Morpholino-2,2-tetramethylenchroman
4-(2,6-Dimethylmorpholino)-2,2-tetramethylenchroman
4-Piperazino-2,2-tetramethylenchroman
4-(4-Methylpiperazino)-2,2-tetramethylenchroman
4-(4-Phenylpiperazino)-2,2-tetramethylenchroman
4-(4-Benzylpiperazino)-2,2-tetramethylenchroman
4-(2-Diäthylaminoäthyl)-amino-2,2-tetramethylenchroman
4-(3-Dimethylaminopropyl)-amino-2,2-tetramethylenchroman
4-Benzylamino-2,2-pentamethylenchroman
4-Benzylamino-2,2-pentamethylen-6-methoxychroman
4-Benzylamino-2,2-pentamethylen-7-methoxychroman
4-Benzylamino-2-methyl-2-nonyl-7-hydroxy-chroman
4-Benzylamino-2,2-tetramethylenchroman
4-Benzylamino-8-methoxy-2,2-tetramethylenchroman
4-Benzylamino-7,8-dimethoxy-2,2-tetramethylenchroman
4-Benzylamino-6,7-dimethoxy-2,2-tetramethylenchroman
4-Benzylamino-2,3,6-trimethyl-chroman
4-Benzylamino-7-benzyloxy-2,2-tetramethylenchroman
4-Benzylamino-2,2-dimethyl-6,8-dichlorchroman
4-Benzylamino-2,2-tetramethylen-6-chlor-8-methylchroman
4-Benzylamino-2,2-pentamethylen-6-chlor-8-methylchroman
4-Benzylamino-2,2-tetramethylen-6-chlorchroman
4-Benzylamino-2,2-tetramethylen-7-methylchroman
4-Dimethylamino-2,3,6-trimethyl-chroman
4-Dimethylamino-2,2-tetramethylen-6-chlor-8-methylchroman
4-Dimethylamino-2,2-pentamethylen-6-chlor-8-methylchroman
4-Dimethylamino-2,2-tetramethylen-6-chlorchroman
4-Dimethylamino-2,2-tetramethylen-7-methylchroman
4-Dimethylamino-2,2-pentamethylenchroman
4-Dimethylamino-2,2-pentamethylen-6-methoxychroman
4-Dimethylamino-2,2-pentamethylen-7-methoxychroman
4-Dimethylamino-2,2-tetramethylenchroman
4-Dimethylamino-8-methoxy-2,2-tetramethylenchroman
4-Dimethylamino-7,8-dimethoxy-2,2-tetramethylenchroman
4-Dimethylamino-6,7-dimethoxy-2,2-tetramethylenchroman
4-Dimethylamino-5,6-benzo-2,2-tetramethylenchroman
4-Dimethylamino-7,8-benzo-2,2-tetramethylenchroman
4-Phenäthylamino-2,2-pentamethylenchroman
4-Phenäthylamino-2,2-pentamethylen-7-methoxychroman
4-Phenäthylamino-2,2-tetramethylenchroman
4-Phenäthylamino-7,8-dimethoxy-2,2-tetramethylenchroman
4-Phenäthylamino-6,7-dimethoxy-2,2-tetramethylenchroman
4-Phenäthylamino-2,2-dimethyl-6,8-dichlorchroman
4-Phenäthylamino-2,2-tetramethylen-6-chlor-8-methylchroman
4-Phenäthylamino-2,2-pentamethylen-6-chlor-8-methylchroman
4-Phenäthylamino-2,2-tetramethylen-6-chlorchroman
4-Phenäthylamino-2,2-tetramethylen-7-methylchroman
4-$\alpha$-Naphthylmethylamino-2,2-tetramethylenchroman
4-$\beta$-Naphthylmethylamino-2,2-tetramethylenchroman
4-$\alpha$-Naphthyläthylamino-2,2-pentamethylenchroman
4-$\alpha$-Naphthyläthylamino-2,2-pentamethylen-6-methoxychroman
4-$\alpha$-Naphthyläthylamino-2,2-pentamethylen-7-methoxychroman

4-$\alpha$-Naphthyläthylamino-2,2-tetramethylenchroman
4-$\alpha$-Naphthyläthylamino-8-methoxy-2,2-tetramethylenchroman
4-$\alpha$-Naphthyläthylamino-7,8-dimethoxy-2,2-tetramethylenchroman
4-$\alpha$-Naphthyläthylamino-5,7-dimethoxy-2,2-tetramethylenchroman
4-$\alpha$-Naphthyläthylamino-2,2-tetramethylen-6-chlor-8-methylchroman
4-$\alpha$-Naphthyläthylamino-2,2-pentamethylen-6-chlor-8-methylchroman
4-$\alpha$-Naphthyläthylamino-2,2-tetramethylen-6-chlorchroman
4-$\alpha$-Naphthyläthylamino-2,2-tetramethylen-7-methylchroman
4-$\beta$-Naphthyläthylamino-2,2-pentamethylenchroman
4-$\beta$-Naphthyläthylamino-2,2-pentamethylen-6-methoxychroman
4-$\beta$-Naphthyläthylamino-2,2-pentamethylen-7-methoxychroman
4-$\beta$-Naphthyläthylamino-2,2-tetramethylenchroman
4-$\beta$-Naphthyläthylamino-8-methoxy-2,2-tetramethylenchroman
4-$\beta$-Naphthyläthylamino-7,8-dimethoxy-2,2-tetramethylenchroman
4-$\beta$-Naphthyläthylamino-6,7-dimethoxy-2,2-tetramethylenchroman
4-$\beta$-Naphthyläthylamino-2,2-tetramethylen-6-chlor-8-methylchroman
4-$\beta$-Naphthyläthylamino-2,2-pentamethylen-6-chlor-8-methylchroman
4-$\beta$-Naphthyläthylamino-2,2-tetramethylen-6-chlorchroman
4-$\beta$-Naphthyläthylamino-2,2-tetramethylen-7-methylchroman
4-$\alpha$-Naphthylmethylamino-2,2-pentamethylenchroman
4-$\alpha$-Naphthylmethylamino-2,2-pentamethylen-6-methoxychroman
4-$\alpha$-Naphthylmethylamino-2,2-pentamethylen-7-methoxychroman
4-$\alpha$-Naphthylmethylamino-2,2-tetramethylenchroman
4-$\alpha$-Naphthylmethylamino-8-methoxy-2,2-tetramethylenchroman
4-$\alpha$-Naphthylmethylamino-7,8-dimethoxy-2,2-tetramethylenchroman
4-$\alpha$-Naphthylmethylamino-6,7-dimethoxy-2,2-tetramethylenchroman
4-$\alpha$-Naphthylmethylamino-2,2-tetramethylen-6-chlor-8-methylchroman
4-$\alpha$-Naphthylmethylamino-2,2-pentamethylen-6-chlor-8-methylchroman
4-$\alpha$-Naphthylmethylamino-2,2-tetramethylen-6-chlorchroman
4-$\alpha$-Naphthylmethylamino-2,2-tetramethylen-7-methylchroman
4-$\beta$-Naphthylmethylamino-2,2-pentamethylenchroman
4-$\beta$-Naphthylmethylamino-2,2-pentamethylen-6-methoxychroman
4-$\beta$-Naphthylmethylamino-2,2-pentamethylen-7-methoxychroman
4-$\beta$-Naphthylmethylamino-2,2-tetramethylenchroman
4-$\beta$-Naphthylmethylamino-8-methoxy-2,2-tetramethylenchroman
4-$\beta$-Naphthylmethylamino-7,8-dimethoxy-2,2-tetramethylenchroman
4-$\beta$-Naphthylmethylamino-6,7-dimethoxy-2,2-tetramethylenchroman
4-$\beta$-Naphthylmethylamino-2,2-tetramethylen-6-chlor-8-methylchroman
4-$\beta$-Naphthylmethylamino-2,2-pentamethylen-6-chlor-8-methylchroman
4-$\beta$-Naphthylmethylamino-2,2-tetramethylen-6-chlorchroman
4-$\beta$-Naphthylmethylamino-2,2-tetramethylen-7-methylchroman

Wirkstoffe der allgemeinen Formel I, in welcher die Reste $R^1$ und $R^2$ gemeinsam mit den angrenzenden C-Atomen carbocyclische oder heterocyclische Ringe bilden, sind neu.

Diese neuen Wirkstoffe werden gemäß den oben erwähnten Herstellungsverfahren erhalten.

Die erfindungsgemäßen, teilweise bekannten, Wirkstoffe der allgemeinen Formel I besitzen, wie bereits erwähnt, die Entwicklung von Insekten und Spinnentieren hemmende Eigenschaften. Sie können daher als Insektizide und Akarizide eingesetzt werden.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werder in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine, wie Calcit, Marmor, Bims, Sepiolith, Dolomit sowie synthetische Granulate aus anorganischen und organischen Mehlen sowie Granulate

aus organischem Material, wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin — Sulfitablaugen und Methylcellulose.

Es können in den Formulierungen Haftmittel, wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe, wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Bei den folgenden Beispielen zur entwicklungshemmenden Wirkung der Wirkstoffe, werden während der gesamten angegebenen Entwicklung der Testtiere die morphologischen Veränderungen, wie zur Hälfte verpuppte Tiere, unvollständig geschlüpfte Larven oder Raupen, defekte Flügel, pupale Kutikula bei Imagines etc., als Mißbildungen gewertet. Die Summe der morphologischen Mißbildungen, zusammen mit den während des Häutungsgeschehens oder der Metamorphose abgetöteten Tiere, wird in Prozent der Gesamtzahl der Versuchstiere bestimmt.

Beispiel A

TABELLE 1

Entwicklungshemmende Wirkung/Laphygma Eier Test

Testtier: Laphygma frugiperda (Eier)

| Wirkstoff Konzentration Beispiel Nr. | Entwicklungshemmende Wirkung bei 0,01% |
|---|---|
| Kontrolle: | 0% |
| Precocen I (bekannt) | 0% |
| 18 | 100% |
| 55 | 100% |

Beispiel B

Entwicklungshemmende Wirkung/Fraßtest

| | |
|---|---|
| Testtiere: | Plutella maculipennis (Raupen im 4. Entwicklungsstadium) 20 Stück |
| | Phaedon cochleariae (Larven im 4. Entwicklungsstadium) 20 Stück |
| Futterpflanzen: | Kohlpflanzen (Brassica oleracea) |
| Lösungsmittel: | 10 Gew.-Teile Aceton |
| Emulgator: | 1 Gew.-Teil Alkylarylpolyglykoläther |

Zur Herstellung einer zweckmäßigen Wirkstoffzubereitung vermischt man 2 Gew.-Teile Wirkstoff mit der angegebenen Menge Lösungsmittel, Emulgator und soviel Wasser, daß eine 1%ige Mischung entsteht, die mit Wasser auf die gewünschte Konzentration verdünnt wird.

Die Testtiere werden mit Blättern der Futterpflanzen, die mit einem gleichmäßigen Spritzbelag der Wirkstoffmischung so überzogen werden, daß die gewählte Wirkstoffkonzentration (Wirkstoffmenge pro Flächeneinheit) auf den Blättern erreicht wird, bis zur Entwicklung der Imago gefüttert.

Zur Kontrolle werden nur mit Lösungsmittel- und Emulgator-Wassergemische der gewählten Konzentration überzogene Blätter verfüttert.

Bei diesem Test werden die aus der folgenden Tabelle ersichtlichen Werte erhalten:

TABELLE 2

Entwicklungshemmende Wirkung/Fraßtest

| Testtiere: | Plutella maculipennis | Phaedon cochleariae |
|---|---|---|
| Wirkstoffe Beispiel Nr. | Entwicklungshemmende 0,01% | Wirkung bei Konzentration 0,01% |
| Kontrolle: | 0% | 0% |
| Precocen I (bekannt) | 60% | 0% |
| 55 | 100% | 100% |
| 72 | 100% | 60% |
| 75 | 100% | 100% |
| 66 | 100% | — |
| 12 | 100% | 100% |
| 19 | 100% | 80% |
| 20 | 100% | 100% |
| 21 | 100% | 80% |
| 27 | 100% | 100% |
| 18 | 100% | — |
| 28 | — | 100% |

Beispiel C

Test mit parasitierenden Räudemilben (Psoroptes cuniculi)

Lösungsmittel: Alkylarylpolyglykoläther

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

Etwa 10—25 Räudemilben (Psoroptes cuniculi) werden in 1 ml der zu testenden Wirkstoffzubereitung gebracht, die in Tablettennester einer Tiefziehverpackung pipettiert wurden. Nach 24 Stunden wird der Abtötungsgrad in Prozent bestimmt. Dabei bedeuten 100%, daß alle und 0%, daß keine Milben angetötet worden sind.

Bei diesem Test werden die aus der folgenden Tabelle ersichtlichen Werte erhalten:

TABELLE 3

Test mit parasitierenden Räudemilben (Psoroptes cuniculi)

| Beispiel-Nr. | Wirkstoffkonz. ppm | Abtötende Wirkung in % |
|---|---|---|
| 12 | 100 | 100 |
| 18 | 100 | 100 |
| 19 | 100 | 100 |
| 20 | 100 | 100 |
| 22 | 100 | 100 |
| 23 | 100 | 100 |
| 33 | 100 | 100 |
| 60 | 100 | 100 |
| 61 | 100 | 100 |
| 62 | 100 | 100 |
| 67 | 100 | 100 |
| 68 | 100 | 100 |
| 69 | 100 | 100 |
| 71 | 100 | 100 |
| 73 | 100 | 100 |

Im folgenden ist die Herstellung einiger erfindungsgemäßer Wirkstoffe angegeben:
1. Verbindungen der allgemeinen Formel I, in welcher X für OH steht:

Beispiel 1

300 g 2,2-Tetramethylenchroman-(4) werden in 1,5 Ltr. Methanol gelöst und innerhalb von zwei Stunden mit 50 g Natriumboranat versetzt. Durch Kühlung hält man die Temperatur unter 35°C. Man hält die Lösung noch 20 Stunden bei 25°C, engt ein, versetzt mit 2 Ltr. Wasser, extrahiert das Produkt zweimal mit Toluol, wäscht die organische Phase mit Wasser, trocknet über Natriumsulfat, filtriert und engt das Filtrat ein. Der Rückstand wird destilliert. Ausbeute: 275 g (92% d. Th.) 2,2-Tetramethylenchromanol-(4), Sdp. 165°C/0,2 Torr, Schmp. 56—58°C.

Beispiel 2

Man verrührt 20 g Lithiumaluminiumhydrid in 1 Ltr. Äther, tropft eine Lösung von 200 g 2,2-Tetramethylenchromanon-(4) in 500 ml Äther innerhalb von 1 Stunde bei unter 20°C zu und laßt 24 Stunden bei 25°C nachrühren. Anschließend versetzt man tropfenweise mit 20 ml Wasser und 60 ml 15-%iger Kalilauge, rührt 2 Stunden nach, saugt ab, engt das Filtrat ein und destilliert. Es werden 183 g (91% d. Th.) des gleichen Produktes wie in Beispiel 1 a erhalten.

Beispiel 3

230 g 2,2-Tetramethylenchromanon-(4) werden in 1 Ltr. Methanol gelöst und in Gegenwart von 30 g Raney-Nickel 6 Stunden bei einem Wasserstoffdruck von 150 atü bei 120—140°C hydriert. Das Reaktionsgemisch wird filtriert, das Filtrat eingeengt und destilliert, wobei man 217 g (93% d. Th.) des gleichen Produktes wie in Beispiel 1 a erhält.

Beispiel 4

Man arbeitet wie in Beispiel 1 a, aber mit Äthanol als Lösungsmittel, und erhält das gleiche Chromanol in 89-%iger Ausbeute.

Die in der Tabelle zusammengefaßten Chromanole wurden analog Beispiel 1 a hergestellt. Unter $R_1$ bis $R_8$ sind die von Wasserstoff abweichenden Substituenten aufgeführt.

TABELLE

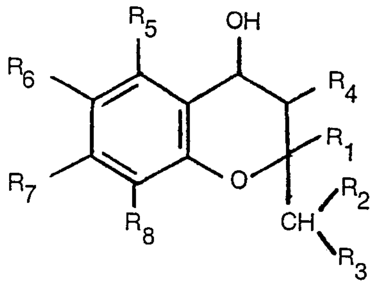

| Beispiel | $R_1$ bis $R_8$ | Ausbeute | Sdp/Torr(Schmp.) |
|---|---|---|---|
| 5 | $R_1+R_2=-(CH_2)_3-$, $R_6=Cl$ | 99% | (118–120°C) |
| 6 | ,, , $R_7=CH_3O$ | 98% | 160°C/0,1 |
| 7 | $R_2=R_3=CH_3$ , $R_4=C_6H_5$, $R_7=CH_3O$ | 98% | (∼40°C) |
| 8 | $R_1+R_2=-(CH_2)_4-$ | 99% | 150°C/0,2 |
| 9 | $R_1+R_2=-CH_2-CH_2-N(CH_3)-CH_2-$ | 77% | (123–125°C) |
| 10 | $R_1+R_2=-(CH_2)_4-$, $R_7=C_6H_5CH_2O-$ | 95% | (113–115°C) |
| 11 | $R_2=R_3=CH_3$, $R_4=C_6H_5$, $R_6=CH_3$ | 98% | (132–133°C) |
| 12 | $R_2=n\text{-}C_5H_{11}$ | 92% | ( 50–52°C) |
| 13 | $R_2=C_6H_5-CH_2$ | 99% | ( 44–45°C) |
| 14 | $R_1=-CH_2CH_2CH_2-N(C_2H_5)_2$ | 77% | 160–5°C/9,1 |
| 15 | $R_1= -CH_2-CH_2-$ 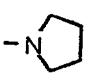 | 51% | (100–101°C) |
| 16 | $R_1+R_2= -(CH_2)_5-$, $R_6 = Cl$ | 79% | (82–83°C) |
| 17 | $R_2= (-CH_2)_3-N(C_2H_5)_2$, $R_7=CH_3O$ | 88% | 185°C/0,1 |
| 18 | $R_1= -CH_2-CH_2-CH=C(CH_3)_2$ | 76% | 160°C/0,1 |
| 19 | $R_2=R_3=CH_3$, $R_6=R_8= Cl$ | 98% | (75–77°C) |
| 20 | $R_1+R_2= -(CH_2)_3-$ | 96% | (95–97°C) |
| 21 | $R_2=n\text{-}C_5H_{11}$, $R_7=CH_3O$ | 65% | 170°C/0,2 |
| 22 | $R_2=n\text{-}C_5H_{11}$, $R_7=CH_3$ | 94% | 70–72°C |
| 23 | $R_2=n\text{-}C_5H_{11}$, $R_6=CH_3O$ | 98% | 66–68°C |
| 24 | $R_1=C_6H_5-CH_2-CH_2$ | 91% | (180°C/0,1) |
| 25 | $R_1=(-CH_2)_3-CH(CH_3)-(CH_2)_2CH=C(CH_3)_2$ | 62% | (190°C/0,1) |
| 26 | $R_1=(-CH_2)_3-N(C_2H_5)_2$, $R_6=Cl$ | 88% | (180°C/0,2) |
| 27 | $R_2+n\text{-}C_5H_{11}$, $R_6=R_8= Cl$ | 57% | (200°C/0,2) |
| 28 | $R_1=n\text{-}C_9H_{19}$, $R_6=R_8= Cl$ | 51% | (190°C/0.1) |
| 29 | $R_1=(-CH_2)_3-N(C_2H_5)_2$, $R_7=C_6H_5-CH_2O$ | 54% | (250°C/0,1) |
| 30 | $R_1=R_7 = CH_3$ | 82% | (115°C/0,2) |
| 31 | $R_1=CH_3$, $R_6=Cl$ | 80% | 101–103°C |

TABELLE (Fortsetzung)

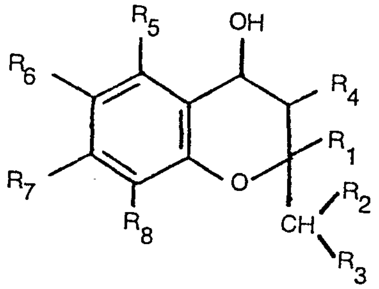

| Beispiel | $R_1$ bis $R_8$ | Ausbeute | Sdp/Torr(Schmp.) |
|---|---|---|---|
| 32 | $R_1+R_2= -(CH_2)_3-$, $R_6=C_6H_5-CH_2O$ | 75% | 74–76°C |
| 33 | $R_1+R_2= -(CH_2)_4-$, $R_6=R_8=Cl$ | 82% | 67–69°C |
| 34 | $R_1=-(CH_2)_2-CH=C(CH_3)_2$, $R_6=R_8=Cl$ | 69% | (165°C/0,1) |
| 35 | $R_1=CH_3$, $R_8=R_8=Cl$ | 95% | (145°C/0,15) |
| 36 | $R_1=-(CH_2)_3-N(C_2H_5)_2$, $R_6=R_8=Cl$ | 87% | (180°C/0,1) |
| 37 | $R_1+R_2= -(CH_2)_3-$, $R_5=R_7=R_8=CH_3=R_6=OH$ | 81% | >100°C(Zers.) |
| 38 | $R_1=COOH$ | 60% | Oel |
| 39 | $R_1+R_2= -(CH_2)_3-$, $R_7=C_6H_5$ | 81% | 91–93°C |
| 40 | $R_1+R_2= -(CH_2)_3-$, $R_6=CH_3O$ | 93% | (155°C/0,1) |
| 41 | $R_1+R_2= -(CH_2)_3-$, $R_5/R_6$=Benzo | 97% | 168–170°C |
| 42 | $R_1+R_2= -(CH_2)_3-$, $R_7/R_8$= Benzo | 93% | 145–6°C |
| 43 | $R_1+R_2= -(CH_2)_4-$, $R_6=Cl$, $R_8=CH_3$ | 92% | (170°C/0,25) |
| 44 | $R_1+R_2= -(CH_2)_3-$, $R_6=Cl$, $R_8=CH_3$ | 92% | (160°C/0,2) |
| 45 | $R_1+R_2= -(CH_2)_3-$, $R_6=R_8=NO_2$ | 72% | Oel |
| 46 | 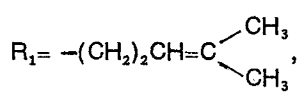, $R_8$ = OH | 42% | Oel |

$R_7$ = 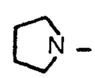

2. Verbindungen der allgemeinen Formel I, in welcher X für —$NR^9R^{10}$ steht:

Beispiel 47

Man löst 15 g 4-Brom-2,2-tetramethylenchroman in 25 ml Toluol und versetzt mit 25 ml Morpholin, worauf die Innentemperatur auf 60°C steigt. Das Gemisch wird nach einem Tag mit 100 ml Toluol und 50 ml 2n-Natronlauge versetzt. Der Toluol-Teil wird mit Wasser gewaschen, über Natriumsulfat getrocknet, filtriert und eingeengt. Man löst den Rückstand in 15 ml Methanol, saugt nach einem Tag ab und erhält 11 g 4-N-Morpholino-2,2-tetramethylenchroman; Schmp. 88—90°C.

Das dabei verwendete 4-Brom-2,2-tetramethylenchroman wird wie folgt erhalten:

Man löst 220 g 2,2-Tetramethylen-4-hydroxychroman in 1 Ltr. Äther, kühlt auf −20°C ab und versetzt innerhalb von 60 Min. mit 220 g Phosphortribromid. Dann entfernt man die Kühlung, beläßt den Ansatz noch einen Tag bei Raumtemperatur, gießt den Ätherteil vorsichtig auf Natriumbicarbonat-Lösung, trennt den wäßrigen Teil ab und wäscht die Äther-Phase zweimal mit Wasser nach. Die Äther-Lösung wird über Natriumsulfat getrocknet, filtriert und bei unter 30°C eingeengt. Es bleiben 251 g 4-Brom-2,2-tetramethylenchroman als Öl zurück.

Beispiel 48

150 g 2,2-Tetramethylenchromanon-(4) werden in 500 ml Tetrahydrofuran gelöst und bei 150°C in Gegenwart von 30 g Raney-Nickel und 300 ml Ammoniak bei einem Druck von 125 atu 5 Stunden mit Wasserstoff hydriert. Nach beendeter Reaktion trennt man den Katalysator ab, engt die Lösung ein und destilliert. Ausbeute: 136 g 4-Amino-2,2-tetramethylenchroman; Sdp. 130°C/0,2 Torr.

Beispiel 49

Man versetzt eine Lösung des in Beispiel 48 erhaltenen Amins in 50 ml Toluol mit 8 ml Essigsäureanhydrid, laßt einen Tag stehen und saugt ab. Es werden 11,5 g 4-Acetylamino-2,2-tetramethylenchroman vom Schmp. 187—189°C erhalten.

Beispiel 50

Man verrührt 6 g Lithiumaluminiumhydrid in 150 ml Glykoldimethyläther, gibt 18 g des nach Beispiel 49 erhaltenen Amids in Portionen zu und erwärmt anschließend 9 Stunden auf Rückflußtemperatur. Nach dem Abkühlen versetzt man langsam bei unter 20°C mit 6 ml Wasser und 18 ml 15%iger Kalilauge, saugt ab, engt das Filtrat ein und destilliert. Man erhält 12 g 4-Aethylamino-2,2-tetramethylenchroman; Sdp. 100°C/0,05 Torr.

Beispiel 51

Man erwärmt eine Lösung von 20 g 2,2-Tetramethylenchromanon-(4) und 11 g Benzylamin in 100 ml Toluol am Wasserabscheider, bis sich kein Wasser mehr abtrennt (4 Stdn.), läßt abkühlen, engt ein, löst den Rückstand in 100 ml Methanol und versetzt mit 4 g Natriumboranat. Nach einem Tag wird der Ansatz auf Wasser gegeben und das Endprodukt mit Toluol extrahiert. Nach dem Trocknen über Natriumsulfat engt man ein und erhält durch Destillation 17 g 2,2-Tetramethylen-4-benzylaminochroman, Sdp. 180°C/0,1 Torr.

Beispiel 52

Eine Lösung von 20 g 4-Hydroxy-2,2-tetramethylenchroman und 15 ml Triäthylamin in 100 ml Toluol wird bei 20°C mit 12 g Methansulfonsäurechlorid in ca. 10 Min. versetzt. Nach einem Tag gießt man auf Wasser, trennt die Toluolphase ab, trocknet über Natriumsulfat, filtriert und engt das Filtrat bei unter 30°C ein. Der Rückstand wird mit 30 ml Allylamin versetzt. Nach einem Tag gießt man auf 100 ml 2n—NaOH, trennt den Toluol-Teil ab, trocknet über Natriumsulfat, filtriert, engt ein und destilliert. Man erhält 13 g 4-Allyl-amino-2,2-tetramethylenchroman; Sdp. 135°C/0,1 Torr.

Beispiel 53

Man verrührt 8 g Lithiumaluminiumhydrid in 300 ml Tetrahydrofuran, gibt bei unter 30°C portionsweise 40 g 4-Oximino-2,2-tetramethylenchroman zu, erhitzt 3 Stdn. auf Rückflußtemperatur und arbeitet analog Beispiel 50 auf. Es werden 29 g 4-Amino-2,2-tetramethylenchroman erhalten; Sdp. 120°C/0,1 Torr.

Beispiel 54

Man löst 35 g 2,2-Pentamethylen-4-amino-6-methoxychroman in 150 ml Methanol, gibt 25 ml einer 70%igen Lösung von Formaldehyd in Methanol und 7 g Raney-Nickel zu und hydriert 4 Stdn. bei 110°C unter einem Wasserstoffdruck von 150 atü. Nach dem Abkühlen filtriert man ab, engt das Filtrat ein und erhält durch Destillation 27 g 2,2-Pentamethylen-4-dimethylamino-6-methoxychroman; Sdp. 135°C/0,1 Torr. Durch Versetzen mit 150 ml 2n—HCl erhält man 28 g des Hydrochlorids; Schmp. 243—245°C.

In der folgenden Tabelle sind für die Reste $R^1$ bis $R^{10}$ diejenigen Reste aufgeführt, die von Wasserstoff abweichen.

Die unter Verfahren angegebene arabische Zahl bezieht sich auf dasjenige Beispiel, analog dem die betreffende Verbindung hergestellt wurde.

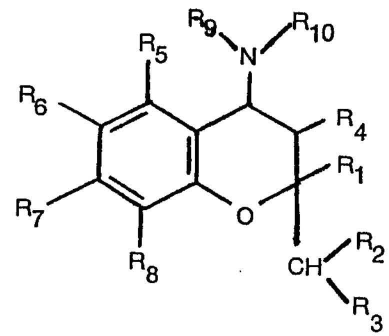

| Bsp.-Nr. | $R_1$ bis $R_{10}$ | | | Verfahren | Sdp./Torr (Fp) |
|---|---|---|---|---|---|
| 55 | $R_1 + R_2 = -(CH_2)_3-$ | | $R_9=R_{10} = CH_3$ | 54 | (Fp 220–1°C) als Hydrochlorid |
| 56 | $R_1 + R_2 = -(CH_2)_4-$ | $R_7 = CH_3O$, | $R_9=R_{10}= CH_3$ | 54 | (Fp 192–4°C) als Hydrochlorid |
| 57 | $R_1 + R_2 = -(CH_2)_6-$ | | $R_9=R_{10} = CH_3$ | 54 | (Fp 195–7°C) als Hydrochlorid |
| 58 | $R_2 = R_3$..................... = | | $R_9=R_{10} = CH_3$ | 54 | 175–80°C/0,1 |
| 59 | $R_1 + R_2 = -(CH_2)_3-$ | | $R_9=R_{10} = n\text{-}C_3H_7$ | 52 | 150°C /0,2 |
| 60 | $R_1 + R_2 = -(CH_2)_3-$ | | $R_9=R_{10} = -(CH_2)_4-$ | 47 | 140°C /0,1 |
| 61 | $R_1 + R_2 = -(CH_2)_3-$ | | $R_9=C_6H_5-CH_2-$ | 47 | 180°C /0,1 |
| 62 | $R_1 + R_2 = -(CH_2)_3-$ | | $R_9=CH_2=CH-CH_2-$ | 47 | 130°C /0,05 |
| 63 | $R_1 + R_2 = -(CH_2)_3-$ | | $R_9$=γ-Pyridylmethyl | 47 | 150°C /0,2 |
| 64 | $R_1 + R_2 = -(CH_2)_3-$ | | $R_9=C_6H_5-CH_2-CH_2$ | 47 | 210°C /0,3 |
| 65 | $R_1 + R_2 = -CH_2-CH_2-N(CH_3)-CH_2-$ | | | | (123°C) |
| 66 | $R_2 = R_3 = CH_3$ | | | | 115°C /0,2 |
| 67 | $R_1 + R_2 = -(CH_2)_3$ | | $R_9 = CH_3O-CH_2-CH_2-$ | 47 | 140°C /0,1 |
| 68 | $R_1 + R_2 = -(CH_2)_3$ | | $R_9 = n\text{-}C_6H_{13}$ | 52 | 150°C /0,1 |
| 69 | $R_1 + R_2 = -(CH_2)_3-$ | | $R_9=R_1$ = Allyl | 47 | 130°C /0,1 |
| 70 | $R_1 + R_2 = -(CH_2)_3-$ | | $R_9 = (CH_3)_2N-(CH_2)_3-$ | 47 | 150°C /0,06 |
| 71 | $R_1 + R_2 = -(CH_2)_3-$ | | $R_9 = n\text{-}C_4H_9$ | 52 | 155°C /0,3 |
| 72 | $R_1 + R_2 = -(CH_2)_3-$ | | $R_9 = C_6H_5-$ | 47 | (85–78°C) |
| 73 | $R_1 + R_2 = -(CH_2)_3-$ | | $R_9 = C_4H_9-CH(C_2H_5)-CH_2-$ | 47 | 170°C /0,1 |

| Bsp.-Nr. | $R_1$ bis $R_{10}$ | | Verfahren | Sdp./Torr (Fp.) |
|---|---|---|---|---|
| 74 | $R_1 + R_2 = -(CH_2)_3-$ | $R_6 = R_8 = Cl$<br>$R_9 = C_4H_9-CH(C_2H_5)-CH_2-$ | 47 | 200 °C / 0,1 |
| 75 | $R_1 + R_2 = -(CH_2)_3-$ | $R_6 = R_8 = Cl$<br>$R_9$ = Allyl | 47 | 160 °C / 0,1 |
| 76 | $R_1 + R_2 = -(CH_2)_3-$ | $R_6 = R_8 = Cl$<br>$R_9 = C_6H_5-CH_2-$ | 51 | (79–81 °C) |
| 77 | $R_1 + R_2 = -(CH_2)_3$ | $R_6 = R_8 = Cl$<br>$R_9 + R_{10} = -(CH_2)_4-$ | 47 | (80–82 °C) |
| 78 | $R_1 + R_2 = -(CH_2)_3-$ | $R_6 = R_8 = Cl$<br>$R_9 = CH_3O-CH_2-CH_2-$ | 47 | 165 °C / 0,1 |
| 79 | $R_1 + R_2 = -(CH_2)_3-$ | $C_7 = Cl$, $R_9 = C_4H_9-CH(C_2H_5)-CH_2-$ | 47 | 190 °C / 0,05 |
| 80 | $R_1 + R_2 = -(CH_2)_3-$ | $R_9 = (C_2H_5)_2N-CH_2-CH_2-$ | 47 | 150 °C / 0,1 |
| 81 | $R_1 + R_2 = -(CH_2)_3-$ | $R_9 = R_{10} = (CH_3)_2N-(CH_2)_3-$ | 47 | 240 °C / 0,1 |
| 82 | $R_1 + R_2 = -(CH_2)_3-$ | $R_9 + R_{10} = -CH_2-CH_2-N(CH_3)-(CH_2)_2-$ | 47 | (83–85 °C) |
| 83 | $R_1 + R_2 = -(CH_2)_3-$ | $R_9$ = α-Naphthylmethyl | 47 | 220 °C / 0,1 |
| 84 | $R_1 + R_2 = -(CH_2)_3-$ | $R_6 = Cl$ $R_9 = C_6H_5-CH_2-CH_2$ | 50 | 200 °C / 0,1 |

| Bsp.-Nr. | $R_1$ bis $R_{10}$ | | Verfahren | Sdp./Torr (Fp) |
|---|---|---|---|---|
| 85 | $R_1 + R_2 = -(CH_2)_4-$ | $R_6 = Cl$ | | |
| | | $R_8 = CH_3$ $R_9 = C_6H_5-CH_2-CH_2-$ | 47 | 245°C / 0,1 |
| 86 | $R_1 + R_2 = -(CH_2)_4-$ | $R_6 = Cl$ | | |
| | | $R_8 = CH_3$ $R_9$ = Benzyl | 47 | 210°C / 0,1 |
| 87 | $R_1 + R_2 = (CH_2)_4-$ | $R_6 = Cl$ | | |
| | | $R_8 = CH_3$ $R_9$ = α-Naphthylmethyl | 47 | (112°C) |
| 88 | $R_1 + R_2 = -(CH_2)_3-$ | $R_6 = Cl$ | | |
| | | $R_8 = CH_3$ $R_9$ = Phenäthyl | 50 | 210°C./ 0,1 |
| 89 | $R_1 + R_2 = -(CH_2)_3-$ | $R_6 = Cl$ $R_8 = CH_3$ $R_9$ = Benzyl | 51 | 200°C / 0,1 |
| 90 | $R_1 + R_2 = -(CH_2)_3-$ | $R_6 = Cl$ | | |
| | | $R_8 = CH_3$ $R_9$ = α-Naphthylmethyl | 47 | (98–100°C) |
| 91 | $R_1 + R_2 = -(CH_2)_3-$ | $R_6 = CH_3O/R_9$ = α-Naphthylmethyl | 47 | 240°C / 0,1 |
| 92 | $R_1 + R_2 = -(CH_2)_3-$ | $R_6 = CH_3O/R_9$ = Phenäthyl | 47 | 200°C / 0,1 |
| 93 | $R_1 + R_2 = -(CH_2)_3-$ | $R_6 = CH_3O/R_9$ = Benzyl | 47 | 190°C / 0,1 |

| Bsp.-Nr. | $R_1$ bis $R_{10}$ | | | Verfahren | Schmp. als Hydrochlorid |
|---|---|---|---|---|---|
| 94 | $R_1 + R_2 = -(CH_2)_3-$ | $R_6 = CH_3$, | $R_9$ = Benzyl | 47 | (230–1 °C) |
| 95 | $R_1 + R_2 = -(CH_2)_3-$ | $R_6 = CH_3$ | $R_9$ = Phenylathyl | 47 | (201–2 °C) |
| 96 | $R_1 + R_2 = -(CH_2)_3-$ | $R_6 = CH_3$, | $R_9$ = $\alpha$-Naphthylmethyl | 47 | (216–8 °C) |
| 97 | $R_1 + R_2 = -(CH_2)_3-$ | $R_7 = CH_3$, | $R_9$ = Benzyl | 47 | (204–6 °C) |
| 98 | $R_1 + R_2 = -(CH_2)_3-$ | $R_7 = CH_3$, | $R_9$ = Phenylathyl | 47 | (201–3 °C) |
| 99 | $R_1 + R_2 = -(CH_2)_3-$ | $R_7 = CH_3$, | $R_9$ = $\alpha$-Naphthylmethyl | 47 | (208–10 °C) |
| 100 | $R_1 + R_2 = -(CH_2)_3-$ | $R_6$ = Cl, | $R_9$ = Benzyl | 47 | (204–6 °C) |
| 101 | $R_1 + R_2 = -(CH_2)_3-$ | $R_6$ = Cl, | $R_9$ = $\alpha$-Naphthylmethyl | 47 | (229–33 °C) |
| 102 | $R_1 + R_2 = -(CH_2)_4-$ | | $R_9$ = Benzyl | 47 | (207–9 °C) |
| 103 | $R_1 + R_2 = -(CH_2)_4-$ | | $R_9$ = Phenylathyl | 47 | (198–9 °C) |
| 104 | $R_1 + R_2 = -(CH_2)_4-$ | | $R_9$ = $\alpha$-Naphthylmethyl | 47 | (216–8 °C) |

**Patentansprüche**

1. Insektizide und akarizide Mittel, gekennzeichnet durch einen Gehalt an Chromanderivaten der allgemeinen Formel I

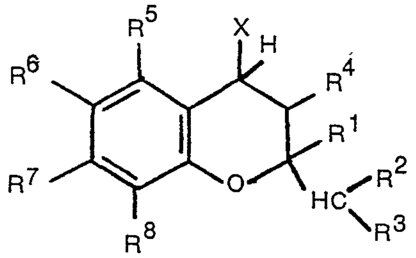

(I)

in welcher

$R^1$ bis $R^4$ gleich oder verschieden sind und für Wasserstoff, Carboxyl oder für Alkyl, Alkenyl oder Alkoxycarbonyl mit jeweils bis zu 18 Kohlenstoffatomen, Cycloalkyl mit 3 bis 18 Kohlenstoffatomen, Aryl mit 6 bis 14 Kohlenstoffatomen oder Aralkyl mit 7 bis 18 Kohlenstoffatomen stehen, wobei diese Reste durch Halogen, Cyano, $C_{1-6}$-Alkyl, $C_{1-6}$-Alkoxy, $C_{2-6}$-Alkoxycarbonyl, $C_{3-6}$-Alkoxycarbonylalkyl, Amino, $C_{1-6}$-Alkylamino, $C_{2-6}$-Dialkylamino, Phenyl oder Carboxyl substituiert sein können und

$R^2$ zusätlich für Amino, Pyrrolidinyl, Piperidinyl und Dialkylamino mit bis zu 18 Kohlenstoffatomen steht und

$R^1$ und $R^2$ gemeinsam mit den angrenzenden Kohlenstoffatomen carbocyclische Ringe der Reihe Cyclopropan, Cyclopentan, Cyclohexan, Cycloheptan, Cyclooctan, Cyclononan, Cyclodecan, Cycloundecan, Cyclododecan, Cyclohexen, Cycloocten, Cyclododecen und Tetralin oder heterocyclische Ringe der Reihe Piperidin, Pyrrolidin, Tetrahydrofuran, Tetrahydropyran, Tetrahydrothiopyran und $-CH_2-CH_2-N(CH_3)-CH_2-$ bilden können und

$R^5$ bis $R^8$ gleich oder verschieden sind und für Wasserstoff, Halogen, Hydroxy, Nitro, Cyano, Amino und Carboxy oder für Alkyl, Alkenyl, Alkoxy, Alkoxycarbonyl, Alkylamino, Dialkylamino mit jeweils bis zu 18 Kohlenstoffatomen, Pyrrolidinyl oder Piperidinyl, Cycloalkyl mit 3 bis 18 Kohlenstoffatomen, Aryl oder Aryloxy mit 6 bis 14 Kohlenstoffatomen und Aralkyl oder Aralkoxy mit 7 bis 18 Kohlenstoffatomen, wobei diese Reste substituiert sein können durch Halogen, $C_1-C_6$-Alkyl, $C_1-C_6$-Alkoxy, $C_2-C_6$-Alkoxycarbonyl, $C_3-C_6$-Alkoxycarbonylalkyl, Amino, $C_1-C_6$-Alkylamino und $C_2-C_6$-Dialkylamino, Phenyl oder Carboxyl oder für Formylamino, Acetylamino, Propionylamino, Valeroylamino und Benzoylamino stehen und

wobei 2 der Reste $R^5$ bis $R^8$, die in o-Stellung zueinander stehen, auch für Tetramethylen oder den Rest $-CH=CH-CH=CH-$ stehen können und

X für OH oder den Rest $-NR^9R^{10}$ steht, wobei

$R^9$ und $R^{10}$ gleich oder verschieden sind und für Wasserstoff, Alkyl oder Alkenyl mit bis zu 18 Kohlenstoffatomen, Cycloalkyl mit 3 bis 18 Kohlenstoffatomen, Aryl mit 6 bis 14 Kohlenstoffatomen, Aralkyl mit 7 bis 18 Kohlenstoffatomen oder gemeinsam mit dem Stickstoffatom, an welches sie gebunden sind, einen Pyrrolidin-, Piperidin-, Morpholin-, N-Methylpiperazin-, N-Phenylpiperazin-Ring bilden sowie die Salze der Verbindungen, in denen

X für den Rest $-NR^9R^{10}$ steht.

2. Verfahren zur Herstellung insektizider und akarizider Mittel, dadurch gekennzeichnet, daß man Chromanderivate der allgemeinen Formel I in Anspruch 1 mit Streckmitteln und/oder oberflächenaktiven Mitteln vermischt.

3. Verwendung von Chromanderivaten der allgemeinen Formel I in Anspruch 1 zur Bekämpfung von Insekten oder Spinnentieren.

4. Neue Chromanderivate der allgemeinen Formel I in Anspruch 1, in welcher die Reste $R^1$ und $R^2$ gemeinsam mit den daran angrenzenden C-Atomen carbocyclische Ringe der Reihe Cyclopropan, Cyclopentan, Cyclohexan, Cycloheptan, Cyclooctan, Cyclononan, Cyclodecan, Cycloundecan, Cyclododecan, Cyclohexen, Cycloocten, Cyclododecen und Tetralin oder heterocyclische Ringe der Reihe Piperidin, Pyrrolidin, Tetrahydrofuran, Tetrahydropyran, Tetrahydrothiopyran und N-Methylpiperidin bilden.

## Claims

1. Insecticidal and acaricidal agents, characterised in that they contain chromane derivatives of the general formula I

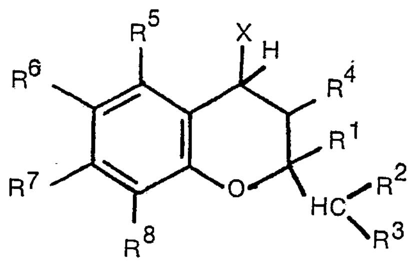

(I)

in which

$R^1$ to $R^4$ are identical or different and represent hydrogen, carboxyl or alkyl, alkenyl or alkoxycarbonyl with in each case up to 18 carbon atoms, cycloalkyl with 3 to 18 carbon atoms, aryl with 6 to 14 carbon atoms or aralkyl with 7 to 18 carbon atoms, it being possible for these radicals to be substituted by halogen, cyano, $C_{1-6}$-alkyl, $C_{1-6}$-alkoxy, $C_{2-6}$-alkoxycarbonyl, $C_{3-6}$-alkoxycarbonylalkyl, amino, $C_{1-6}$-alkylamino, $C_{2-6}$-dialkylamino, phenyl or carboxyl and

$R^2$ additionally represents amino, pyrrolidinyl, piperidinyl and dialkylamino with up to 18 carbon atoms and

$R^1$ and $R^2$ can, conjointly with the adjoining carbon atoms, form carbocyclic rings of the series comprising cyclopropane, cyclopentane, cyclohexane, cycloheptane, cyclooctane, cyclononane, cyclodecane, cycloundecane, cyclododecane, cyclohexene, cyclooctene, cyclododecene and tetralin or heterocyclic rings of the series comprising piperidine, pyrrolidine, tetrahydrofuran, tetrahydropyrane, tetrahydrothiopyrane and $—CH_2—CH_2—N(CH_3)—CH_2—$ and

$R^5$ and $R^8$ are identical or different and represent hydrogen, halogen, hydroxy, nitro, cyano, amino and carboxyl or alkyl, alkenyl, alkoxy, alkoxycarbonyl, alkylamino, dialkylamino with in each case up to 18 carbon atoms, pyrrolidinyl or piperidinyl, cycloalkyl with 3 to 18 carbon atoms, aryl or aryloxy with 6 to 14 carbon atoms and aralkyl or aralkoxy with 7 to 18 carbon atoms, it being possible for these radicals to be substituted by halogen, $C_1—C_6$-alkyl, $C_1—C_6$-alkoxy, $C_2—C_6$-alkoxycarbonyl, $C_3—C_6$-alkoxycarbonylalkyl, amino, $C_1—C_6$-alkylamino and $C_2—C_6$-dialkylamino, phenyl or carboxyl, or represent formylamino, acetylamino, propionylamino, valeroylamino and benzoylamino and two of the radicals $R^5$ and $R^8$, which are in the o-position to one other, can also represent tetramethylene or the radical $—CH = CH—CH = CH—$ and

X represents OH or the radical $—NR^9R^{10}$, wherein

$R^9$ and $R^{10}$ are identical or different and represent hydrogen, alkyl or alkenyl with up to 18 carbon atoms, cycloalkyl with 3 to 18 carbon atoms, aryl with 6 to 14 carbon atoms, aralkyl with 7 to 18 carbon atoms or conjointly with the nitrogen atom to which they are bound form a pyrrolidine, piperidine, morpholine, N-methylpiperazine or N-phenylpiperazine ring, and the salts of the compounds, in which

X represents the radical $—NR^9R^{10}$.

2. Process for the preparation of insecticidal and acaricidal agents, characterised in that chromane derivatives of the general formula I in Claim 1 are mixed with extenders and/or surface-active agents.

3. Use of chromane derivatives of the general formula I in Claim 1 for combating insects or arachnidae.

4. New chromane derivatives of the general formula I in Claim 1, in which the radicals $R^1$ and $R^2$ conjointly with the adjoining C-atoms form carbocyclic rings of the series comprising cyclopropane, cyclopentane, cyclohexane, cycloheptane, cyclooctane, cyclononane, cyclodecane, cycloundecane, cyclododecane, cyclohexene, cyclooctene, cyclododecene and tetralin or heterocyclic rings of the series comprising piperidine, pyrrolidine, tetrahydrofuran, tetrahydropyrane, tetrahydrothiopyrane and N-methylpiperidine.

## Revendications

1. Produits insecticides et acaricides caractérisés en ce qu'ils contiennent des dérivés du chromanne de formule générale I

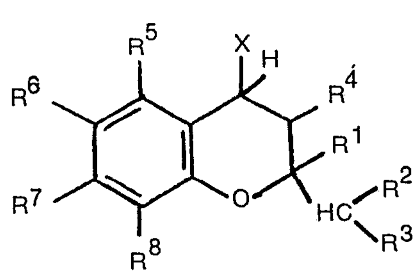

(I)

dans laquelle

$R^1$ à $R^4$, identiques ou différents, représentent l'hydrogène, des groupes carboxyle ou des groupes alkyle, alcényle ou alcoxycarbonyle contenant chacun jusqu'à 18 atomes de carbone, cycloalkyle contenant 3 à 18 atomes de carbone, aryle contenant 6 à 14 atomes de carbone ou aralkyle contenant 7 à 18 atomes de carbone, ces restes pouvant être substitués par des halogènes, des groupes cyano, alkyle en $C_1$—$C_6$, alcoxy en $C_1$—$C_6$, alcoxycarbonyle en $C_2$—$C_6$, alcoxycarbonylalkyle en $C_3$—$C_6$, amino, alkylamino en $C_1$—$C_6$, dialkylamino en $C_2$—$C_6$, phényle ou carbonyle et

$R^2$ représente en outre un groupe amino, pyrrolidinyle, pipéridinyle ou dialkylamino contenant jusqu'à 18 atomes de carbone,

$R^1$ et $R^2$ pouvant former ensemble et avec les atomes de carbone voisins des noyaux carbocycliques de la série du cyclopropane, du cyclopentane, du cyclohexane, du cycloheptane, du cyclooctane, du cyclononane, du cyclodécane, du cycloundécane, du cyclododécane, du cyclohexène, du cyclooctène, du cyclododécène et de la tétraline ou bien des noyaux hétérocycliques de la série de pipéridine, de la pyrrolidine, du tétrahydrofuranne, du tétrahydropyranne, du tétrahydrothiopyranne et $—CH_2—CH_2—N(CH_3)—CH_2—$ et

$R^5$ à $R^8$, identiques ou différents, représentent l'hydrogène, des halogènes, des groupes hydroxy, nitro, cyano, amino et carboxy ou des groupes alkyle, alcényle, alcoxy, alcoxycarbonyle, alkylamino, dialkylamino contenant chacun jusqu'à 18 atomes de carbone, pyrrolidinyle ou pypéridinyle, cycloalkyle contenant 3 à 18 atomes de carbone, aryle ou aryloxy contenant 6 à 14 atomes de carbone et aralkyle ou aralcoxy contenant 7 à 18 atomes de carbone, ces restes pouvant être substitués par des halogènes, des groupes alkyle en $C_1$—$C_6$, alcoxy en $C_1$—$C_6$, alcoxycarbonyle en $C_2$—$C_6$, alcoxycarbonylalkyle en $C_3$—$C_6$, amino, alkylamino en $C_1$—$C_6$ et dialkylamino en $C_2$—$C_6$, phényle ou carboxyle, ou des groupes formylamino, acétylamino, propionylamino, valéroylamino et benzoylamino et deux des restes $R^5$ à $R^8$, en position mutuelle ortho, peuvent également former le groupe tétraméthylène ou le reste $—CH = CH—CH = CH—$ et

X représente OH ou le reste $—NR^9R^{10}$,

$R^9$ et $R^{10}$, identiques ou différents, représentant l'hydrogène, des groupes alkyle ou alcényle contenant jusqu'à 18 atomes de carbone, cycloalkyle contenant 3 à 18 atomes de carbone, aryle contenant 6 à 14 atomes de carbone, aralkyle contenant 7 à 18 atomes de carbone, ou formant en commun et avec l'atome d'azote sur lequel ils sont fixés un cycle pyrrolidine, pipéridine, morpholine, N-méthylpipérazine, N-phénylpipérazine, ainsi que les sels des composés dans lesquels

X représente le reste $—NR^9R^{10}$.

2. Procédé de préparation de produits insecticides et acaricides, caractérisé en ce que l'on mélange des dérivés du chromanne de formule générale I selon la revendication 1 avec des diluants et/ou agents tensioactifs.

3. Utilisation des dérivés du chromanne de formule générale I selon la revendication 1 dans la lutte contre les insectes ou les acariens.

4. Nouveaux dérivés du chromanne de formule générale I selon la revendication 1 dans laquelle les restes $R^1$ et $R^2$ forment ensemble et avec les atomes de carbone voisins des noyaux carbocycliques de la série du cyclopropane, du cyclopentane, du cyclohexane, du cycloheptane, du cyclooctane, du cyclononane, du cyclodécane, du cycloundécane, du cyclododécane, du cyclohexène, du cyclooctène, du cyclododécène et de la tétraline ou des noyaux hétérocycliques de la série de la pipéridine, de la pyrrolidine, du tétrahydrofuranne, du tétrahydropyranne, du tétrahydrothiopyranne et de la N-méthylpipéridine.